**Europäisches Patentamt**

**European Patent Office**

**Office européen des brevets**

(19)

(11) Numéro de publication: **0 211 792**
**A1**

(12)

# DEMANDE DE BREVET EUROPEEN

(21) Numéro de dépôt: **86460014.3**

(22) Date de dépôt: **18.07.86**

(51) Int. Cl.⁴: **A 61 B 17/16**
**A 61 F 2/46**

(30) Priorité: **19.07.85 FR 8511209**

(43) Date de publication de la demande:
**25.02.87 Bulletin 87/9**

(84) Etats contractants désignés:
**AT BE CH DE GB IT LI LU NL SE**

(71) Demandeur: **FRANCE IMPLANT**
**"La Hucheraie"**
**F-35360 Montauban-de-Bretagne(FR)**

(72) Inventeur: **Masse, André, Abel Jean**
**21, rue Brizeux**
**F-35000 Rennes(FR)**

(72) Inventeur: **Malet, Christian, Jean-Pierre**
**14, rue Sarrette**
**F-75014 Paris(FR)**

(74) Mandataire: **Martin, Jean-Jacques et al,**
**Cabinet REGIMBEAU Corre, Martin, Schrimpf, Warcoin,**
**Ahner 11, rue Franz Heller**
**F-35700 Rennes(FR)**

(54) **Râpe pour la préparation du canal médullaire d'un os destiné à recevoir une prothèse.**

(57) Cette râpe comprend un manche (1) et une lame dentelée
(2) composée de deux parties relativement mobiles (3, 4) ; le
déplacement transversal de la partie (4) par rapport à la partie
(3) est réalisé au moyen d'une vis (5), permettant au chirurgien de modifier la largeur de la lame (2) pour qu'elle corresponde à celle de la prothèse qu'il doit mettre en place.
Matériel chirurgical.

EP 0 211 792 A1

./...

Croydon Printing Company Ltd.

FIG.1

"RAPE POUR LA PREPARATION DU CANAL MEDULLAIRE D'UN OS DESTINE A RECEVOIR UNE PROTHESE"

La présente invention concerne une râpe pour la préparation du canal médullaire d'un os destiné à recevoir une prothèse.

Préalablement à la mise en place d'une prothèse dans un os, et notamment d'une prothèse de hanche dans la partie supérieure d'un fémur, le chirurgien doit préparer la cavité médullaire de cet os de manière à lui donner une forme complémentaire de celle de la prothèse. Cette opération est faite à l'aide d'une râpe comprenant un manche de préhension et une lame dentelée dont la forme correspond à celle de la prothèse. Les dents de la lame sont acérées, ce qui leur permet d'enlever par grattage - à la manière d'une lime - l'os spongieux environnant.

On connaît déjà par le document FR-A-2 547 192 une

rāpe fémorale creuse qui est formée de deux parties allongées complémentaires dont la séparation, après l'opération, permet d'enlever les débris d'os qui ont été collectés à l'intérieur de la rāpe.

Il existe, par ailleurs, des instruments médicaux - appelés spéculums - qui sont constitués d'une paire de mâchoires pouvant être écartées l'une de l'autre de manière à dilater certains tissus ; ce type d'instrument, qui est décrit par exemple dans le brevet US-3 835 843 appartient à un domaine technique différent de celui de l'invention.

On sait que les formes et dimensions de la prothèse qui doit être mise en place à l'extrémité d'un os sont sélectionnées en fonction des caractéristiques dimensionnelles et mécaniques de l'os en question et du type de lésion auquel on a affaire. C'est pourquoi le chirurgien doit avoir en permanence à sa disposition une grande quantité de rāpes, de forme et de largeurs différentes. Ainsi, pour une forme donnée de prothèse de la hanche, il est nécessaire de disposer d'une dizaine de rāpes, dont les largeurs en leur partie médiane varient, de millimètre en millimètre, depuis 10 mm jusqu'à 20 mm environ. Ce stock élevé est naturellement coûteux et pose des difficultés de manipulation.

L'invention a pour but de résoudre ce problème en proposant une rāpe du genre mentionné grâce à laquelle il est possible de préparer le canal médullaire d'os destinés à recevoir des prothèses de largeurs différentes.

Ce résultat est obtenu, conformément à l'invention, par le fait que la partie active de la rāpe, en forme

de lame dentelée, est constituée de deux parties allongées complémentaires qui sont mobiles l'une par rapport à l'autre transversalement par rapport à leur direction longitudinale, le déplacement relatif de ces deux parties permettant de faire varier la largeur de cette lame.

Dans un mode de réalisation préférentiel, l'une des parties qui constitue la lame, appelée partie fixe, est solidaire du manche, tandis que l'autre partie est guidée en translation pour coulisser transversalement par rapport à la partie fixe.

Ce coulissement est avantageusement favorisé grâce à la coopération d'une nervure de guidage formée sur l'une des deux parties et d'une rainure ménagée dans l'autre.

Le déplacement relatif des deux parties peut être simplement réalisé à l'aide d'une vis. Celle-ci est par exemple guidée en rotation et immobilisée en translation dans la partie fixe, sa tige filetée s'engageant dans un trou taraudé de la partie mobile. La vis est munie de préférence d'une tête de commande manuelle telle qu'un disque moleté par exemple.

Selon une caractéristique particulièrement intéressante de l'invention, la râpe est avantageusement pourvue d'une échelle graduée adaptée pour repérer la position des deux parties constituant la lame en affichant la largeur de celle-ci.

Selon une autre caractéristique, l'axe du manche de la râpe est incliné par rapport à la direction longitudinale de la lame, ce qui facilite sensiblement l'action de râpage. Dans ce cas, les moyens de montage du manche sur la lame sont avantageusement adaptés pour permettre au manche d'occuper au choix l'une ou l'autre de deux positions décalées angulairement de 180° autour de son propre axe.

D'autres caractéristiques et avantages de l'invention apparaîtront de la description et des dessins annexés qui en présentent un mode de réalisation préférentiel.

La figure 1 est une vue de face, partiellement coupée de la râpe.

La figure 2 est une vue de droite de la partie de lame mobile de la râpe de la figure 1.

La figure 3 est une vue de détail, coupée par le plan III de la figure 1 qui montre les moyens de montage du manche sur la lame.

Les figures 4 et 5 sont des vues de face partielles de la râpe montrant deux largeurs de lame différentes.

La figure 6 est une section de la lame de râpe, suivant le plan horizontal VI de la figure 4.

La râpe qui est représentée sur les figures comprend deux parties principales : un manche 1 destiné à assurer la prise en main de l'instrument par le chirurgien et une lame dentelée 2, de forme allongée, d'épaisseur et de largeur décroissantes en direction de sa pointe.

La longueur de la lame 2 est de l'ordre de 160 mm.

Son épaisseur est de l'ordre de 13 mm à sa base et de 9 mm à sa pointe.

Le manche 1 a une forme légèrement coudée et l'axe $X_1$ de sa partie principale (utile à la préhension) est incliné d'un angle $\alpha$ par rapport à la direction longitudinale $X_2$ de la lame 2. Cet angle $\alpha$ a par exemple une valeur de 35° environ.

La lame 2 est constituée de deux parties : une partie fixe 3, solidaire du manche 1 et une partie mobile 4. Ces parties 3 et 4 sont toutes deux hérissées de dents transversales acérées 33, 43 disposées à leur périphérie à la manière d'écailles. La partie mobile 4 est munie d'une nervure de guidage 40 apte à s'engager dans une rainure complémentaire 30 formée dans la partie fixe 3. Après emboîtement de la nervure 40 dans la rainure 30, la lame 2 formée par ces deux parties 3, 4 a un contour similaire à celui de la prothèse que l'on souhaite mettre en place. Dans l'exemple représenté, il s'agit d'une prothèse de la hanche présentant une pointe effilée et une base arquée plus large.

La base de la partie fixe 3 présente une cavité quasi cylindrique 31 adaptée pour recevoir à coulissement la base 41, en forme de douille complémentaire, de la partie mobile 4. Le déplacement de la douille 41 dans la cavité 31 est réalisé au moyen d'une vis 5. Celle-ci est guidée en translation dans un alésage 32 et immobilisée en translation, par un épaulement 52 et un circlips 6, dans la base de la partie de lame fixe 3. Sa tige filetée 50 pénêtre dans un trou taraudé complémentaire 42 traversant la douille 41. Cette vis possède une tête de commande en forme de disque moleté ou cannelé permettant de la faire tourner aisément à la main.

La nervure 40 est munie, sur l'une au moins de ses faces, et de préférence sur ses deux faces, de repères gravés qui forment une échelle graduée 44. L'index destiné à coopérer avec cette échelle est constitué simplement par le bord interne (rectiligne) 34 de la partie fixe 3.

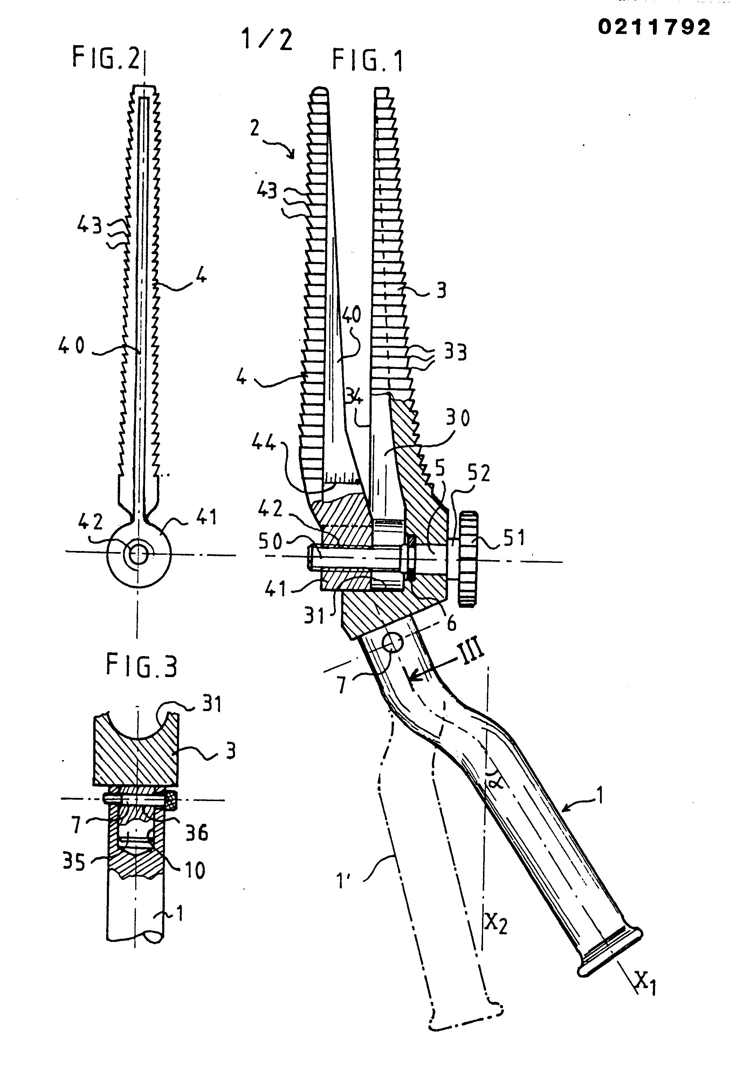

A sa partie supérieure, le manche 1 est pourvu d'un alésage 10 dans lequel s'emboîte un appendice cylindrique 35 de la partie fixe 3. La solidarisation du manche 1 avec l'appendice 35 est réalisée au moyen d'une vis moletée 7 qui s'engage dans un alésage 36 traversant diamètralement cet appendice. Selon la position donnée au manche 1 par rapport à l'alésage 36, il est possible de donner au manche deux orientations différentes, décalées de 180° l'une par rapport à l'autre, comme on le voit à la figure 1 où la deuxième position possible, désignée par la référence 1', a été représentée en traits mixtes.

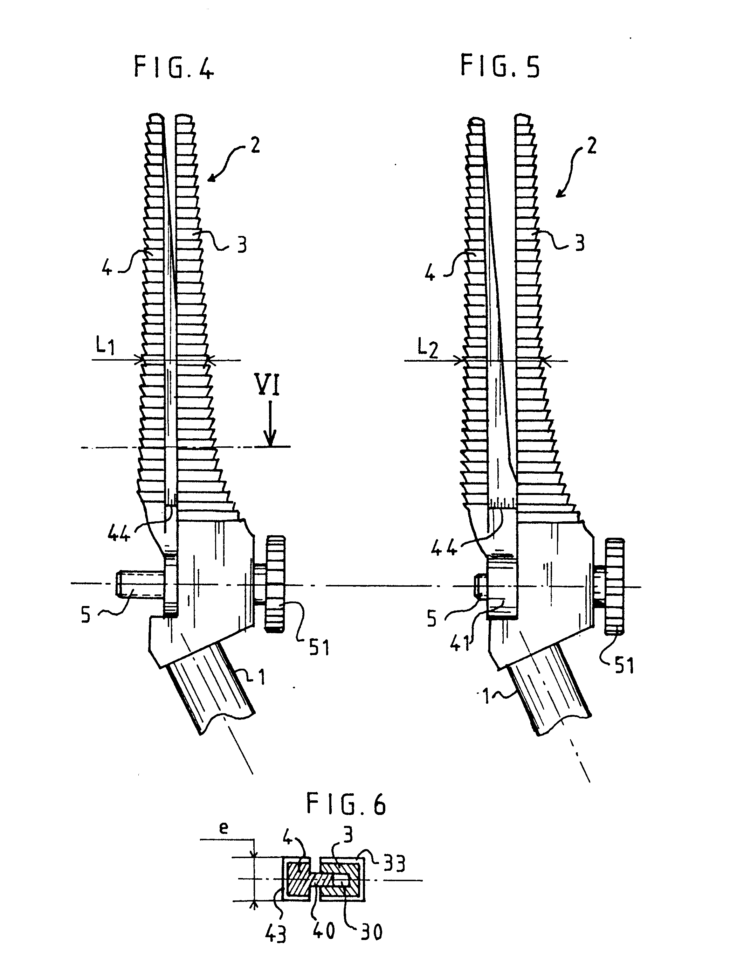

Pour utiliser cette râpe, le chirurgien commence par déterminer la largeur de la prothèse qu'il compte utiliser. Ensuite, en agissant sur le disque moleté 51, il fait tourner dans un sens ou dans l'autre la vis 5 de manière à rapprocher ou au contraire à écarter l'une de l'autre les deux parties 3 et 4. L'échelle graduée 44 le renseigne sur cet ajustage. Les figures 4 et 5 illustrent deux réglages de largeur différentes dont les valeurs sont respectivement L1 et L2 dans la zone médiane de la râpe. Les valeurs de réglage possibles pour L1 et L2 s'inscrivent dans une fourchette avantageusement comprise entre 10 et 20 mm.

Lorsque l'ajustage souhaité a été atteint, le chirurgien utilise cet instrument comme une râpe ordinaire pour préparer le canal médullaire destiné à recevoir la prothèse. Selon la technique opératoire mise en oeuvre, il lui est possible de positionner le manche 1 dans l'une ou l'autre de ses deux positions décalées à 180°.

Les graduations de l'échelle 44 peuvent être directement numérotées en fonction des références des différentes prothèses utilisables, ou dans le cas de l'utilisation d'un dispositif de prothèse conforme à la demande de brevet n° 85 05348 du 5 avril 1985, en fonction des références des différentes barrettes modulaires adaptables sur ce dispositif.

Pour couvrir toutes les largeurs possibles de prothèses d'un type déterminé, il est possible naturellement de prévoir une râpe unique à course de réglage élevée. Dans certains cas, il peut être avantageux, cependant, de prévoir deux râpes à course de réglage plus limitée l'une adaptée aux petites tailles et l'autre aux grandes tailles.

Il va de soi que l'invention n'est pas limitée au mode de réalisation qui vient d'être dévrit, à simple titre d'exemple. Elle en englobe au contraire toutes les variantes.

C'est ainsi, notamment, que le déplacement relatif des deux parties de lame pourrait être réalisé par des moyens différents du système vis-écrou, par exemple par un système pignon-crémaillère ou par un système à pans inclinés (à effet de coin).

Les dents transversales 33, 43 pourraient être remplacées par des picots ou d'autres aspérités aptes à réaliser l'action de râpage souhaitée.

Enfin, divers contours de râpes peuvent naturellement être prévus en fonction du type de prothèse concerné.

REVENDICATIONS

---

1. Râpe pour la préparation du canal médullaire d'un os destiné à recevoir une prothèse, qui comprend un manche et une lame dentelée constituée de deux parties allongées complémentaires, caractérisée par le fait que ces deux parties (3, 4) sont mobiles l'une par rapport à l'autre, transversalement par rapport à leur direction longitudinale, leur déplacement relatif permettant de faire varier la largeur de ladite lame (2).

2. Râpe selon la revendication 1, caractérisée en ce que l'une (3) des parties constituant la lame (2), appelée partie fixe, est solidaire du manche (1), et que l'autre partie (4) est guidée en translation pour coulisser transversalement par rapport à la partie fixe (3).

3. Râpe selon l'une des revendications 1 ou 2, caractérisée en ce que l'une (4) des parties constituant la lame (2) est pourvue d'une nervure de guidage (40) qui s'engage dans une rainure (30) de forme complémentaire ménagée dans l'autre partie (3).

4. Râpe selon l'une des revendications 1 à 3, caractérisée en ce qu'elle comprend une vis (5) adaptée pour assurer le déplacement relatif des deux parties (3) et (4).

5. Râpe selon les revendications 2 et 4 prises en combinaison, caractérisée en ce que la vis (5) est guidée en rotation et immobilisée en translation dans la partie fixe (3), sa tige filetée (50) s'engageant dans un trou taraudé (42) de la partie mobile (4).

6. Râpe selon la revendication 5, caractérisée en ce que la vis (5) est munie d'une tête de commande manuelle, telle qu'un disque moleté (51).

7. Râpe selon l'une des revendications précédentes, caractérisée en ce qu'elle est pourvue d'une échelle graduée (44) adaptée pour repérer la position relative des deux parties (3) et (4) constituant la lame (2) en affichant la largeur de celle-ci.

8. Râpe selon l'une des revendications précédentes, caractérisée en ce que l'axe ($X_1$) de son manche (1) est incliné par rapport à la direction longitudinale ($X_2$) de la lame (2).

9. Râpe selon la revendication 8, caractérisée en ce que les moyens de montage du manche (1) sur la lame (2) sont adaptés pour permettre au manche (1) d'occuper au choix l'une ou l'autre de deux positions décalées angulairement de 180° autour de son propre axe ($X_1$).

FIG.2  1/2  FIG.1

0211792

FIG.3

FIG.4

FIG.5

$L_1$

VI

$L_2$

44

44

5

41

5

51

51

1

1

FIG.6

e

4

3

33

43

40

30

**Office européen des brevets**

**RAPPORT DE RECHERCHE EUROPEENNE**

**0211792**

Numero de la demande

EP 86 46 0014

## DOCUMENTS CONSIDERES COMME PERTINENTS

| Catégorie | Citation du document avec indication. en cas de besoin. des parties pertinentes | Revendication concernée | CLASSEMENT DE LA DEMANDE (Int Cl 4) |
|---|---|---|---|
| Y | FR-A-2 547 192 (LAHILLE)<br>* Page 2, lignes 13-18; figures * | 1 | A 61 B 17/16<br>A 61 F 2/46 |
| A | | 2 | |
| | --- | | |
| Y | US-A-3 835 843 (KARMAN)<br>* Colonne 4, lignes 62-65; figure 3 * | 1 | |
| A | | 4-6 | |
| | --- | | |
| A | US-A-4 466 429 (LOSCHER et al.)<br>* Colonne 3, lignes 14-17,53-55; figures * | 1 | |
| | --- | | DOMAINES TECHNIQUES RECHERCHES (Int. Cl.4) |
| A | US-A-4 350 151 (SCOTT)<br>* Colonne 3, lignes 9-14; figures * | 3 | A 61 B<br>A 61 F<br>A 61 M |
| | --- | | |
| A | FR-A-1 079 467 (REY)<br>* Figure 2; repère G * | 7 | |
| | --- | | |
| A | US-A-4 306 550 (FORTE)<br>* Colonne 2, lignes 44-60; figures * | 8,9 | |
| | ----- | | |

Le present rapport de recherche a ete etabli pour toutes les revendications

| Lieu de la recherche<br>LA HAYE | Date d achèvement de la recherche<br>30-10-1986 | Examinateur<br>GLAS J. |
|---|---|---|